# EUROPEAN PATENT APPLICATION

(11) **EP 4 725 473 A1**
(43) Date of publication of application: **15.04.2026**
(21) Application number: 24819323.7
(22) Date of filing: 04.06.2024
(51) Int. Cl.: A61K 8/19, A61K 8/02, A61K 8/81, A61K 8/86, A61Q 1/00, A61Q 1/10

(54) **AQUEOUS LIQUID COSMETIC**

(30) Priority: 08.06.2023 JP 2023095047; 16.02.2024 JP 2024022036
(71) Applicant: MITSUBISHI PENCIL COMPANY, LIMITED, Tokyo 140-8537 (JP)
(72) Inventor: INOUE Kensuke, Fujioka-shi, Gunma 375-8501 (JP)
(74) Representative: Vossius & Partner Patentanwälte Rechtsanwälte mbB
(86) International application number: PCT/JP2024/020386
(87) International publication number: WO 2024/253094

(57) **Abstract**

An object of the present disclosure is to provide a highly color-developing aqueous liquid cosmetic which is suitable for makeup cosmetics such as an eyeliner, an eyeshadow, and an eyebrow for drawing lines on eyebrows, and in which aggregation and sedimentation of coloring materials hardly occur. An aqueous liquid cosmetic of the present disclosure contains at least a plant-derived pigment, a film-forming agent, a dispersant, and water, wherein the plant-derived pigment is contained in an amount of 1 to 30 mass% with respect to a total amount of the aqueous liquid cosmetic, and an intensity ratio G/D of a D band (1350 cm⁻¹) and a G band (1582 cm⁻¹) in a Raman spectrum of the plant-derived pigment is 0.5 to 10. A liquid cosmetic applicator 10 of the present disclosure is characterized in that the aqueous liquid cosmetic having the above configuration is mounted.

## Description

### Technical Field

The present specification relates to an aqueous liquid cosmetic suitable as makeup cosmetics such as an eyeliner, an eyeshadow, an eyebrow for drawing lines on eyebrows, and a mascara.

### Background Art

In the prior art, black pigments have been used in makeup cosmetics such as an eyeliner, an eyeshadow, an eyebrow for drawing lines on eyebrows, and mascara, and black iron oxide or the like is used as the black pigment to be used (see, for example, Patent Document 1). In the formulation of the black iron oxide, the specific gravity is large, and there have been problems such as a decrease in color strength of drawing lines and low coloration due to sedimentation.

On the other hand, in recent years, an aqueous liquid cosmetic using charcoal and the like has been known as a black pigment in consideration of reduction of environmental load, safety, and the like.

For example, (1) a liquid cosmetic composition containing 50 to 90 mass% of water and 1 to 10 mass% of charcoal having an average particle diameter of 0.05 to 1 µm (see, for example, Patent Document 2); (2) a plant charcoal fine powder in which the average particle diameter is 0.15 to 0.7 µm, a minimum particle diameter is controlled to 0.1 µm or more by pulverization, a carbon content is 96 mass% or more; a cosmetic containing the plant charcoal fine powder (see, for example, Patent Document 3); (3) an aqueous dispersion containing water, at least 22 to 40 mass% of a black pigment, a dispersant which is a polymer having a mass% ratio of 30:1 to 3:1 to the black pigment or a surfactant having an HLB of 10 or more, and a polyhydric alcohol having a mass% ratio of 3:1 to 5:3 to the black pigment, in which the average particle diameter (cumulative average diameter/center diameter) is 340 to 392 nm and a lightness L value is less than 24; and cosmetics (see, for example, Patent Document 4) containing the aqueous dispersion are known.

However, in the aqueous liquid cosmetic and the like containing charcoal with a predetermined particle diameter, plant charcoal fine powder, and the like of Patent Documents 2 to 4 described above, aggregation and sedimentation of coloring materials are suppressed as compared with the known cosmetics using an iron oxide pigment; however, there are still cases where color developability and dispersion stability are insufficient, and in the case of being mounted on a direct-liquid eyeliner, an eyeshadow, and the like, there are still problems such as insufficient discharge, and poor writing properties (applicability), water-resistant fixation after application, and the like.

### Citation List

### Patent Document

Patent Document 1: JP 2014-101298 A (Claims, Examples, etc.)
Patent Document 2: JP 2006-22008 A (Claims, Examples, etc.)
Patent Document 3: JP 2022-27367 A (Claims, Examples, etc.)
Patent Document 4: JP 2023-13339 A (Claims, Examples, etc.)

### Summary of Invention

### Technical Problem

The present disclosure has been made in view of the above known problems and current circumstances, and is intended to solve the problems, and an object of the present disclosure is to provide an aqueous liquid cosmetic which is excellent in color developability and dispersion stability even when a pigment of a plant-derived raw material having a good image in cosmetics is used, can be discharged well even when mounted on a liquid cosmetic applicator for makeup cosmetics such as a direct-liquid eyeliner, an eyeshadow, an eyebrow for drawing lines on eyebrows, and a mascara as well as a cotton-pad-type liquid cosmetic applicator, and also has good writing properties (applicability) and water-resistant fixation after application.

### Solution to Problem

As a result of intensive studies in view of the above-described known problems and the like, the present inventors have found that an aqueous liquid cosmetic of the above object and the like can be obtained by containing at least a predetermined amount of a plant-derived pigment, a film-forming agent, a dispersant, and the like, and also containing a plant-derived pigment having physical properties in a predetermined range, and have completed the present disclosure.

That is, an aqueous liquid cosmetic of the present disclosure contains at least a plant-derived pigment, a film-forming agent, a dispersant, and water, wherein the plant-derived pigment is contained in an amount of 1 to 30 mass% with respect to a total amount of the aqueous liquid cosmetic, and an intensity ratio G/D of a D band (1350 cm⁻¹) and a G band (1582 cm⁻¹) in a Raman spectrum of the plant-derived pigment is 0.5 to 10.

The plant-derived pigment preferably has a BET specific surface area of 20 to 800 m²/g.

The plant-derived pigment preferably further contains a pigment selected from the following group A.

Group A: yellow iron oxide, red iron oxide, black iron oxide, titanium dioxide, Red 226, carmine, iron blue, ultramarine, manganese violet, chromium oxide, and lake pigment.

The film-forming agent is preferably a resin emulsion.

The liquid cosmetic applicator of the present disclosure is characterized in that the aqueous liquid cosmetic having the above configuration is mounted.

### Advantageous Effects of Invention

According to the present disclosure, there is provided an aqueous liquid cosmetic which is excellent in color developability and dispersion stability even when a plant-derived pigment such as plant charcoal is used, can be discharged well even when mounted on an applicator for makeup cosmetics such as a direct-liquid eyeliner, an eyeshadow, an eyebrow for drawing lines on eyebrows, and a mascara as well as a cotton-pad-type liquid cosmetic applicator, can draw a drawing line having high color development, and has good writing properties (applicability) and water-resistant fixation after application.

The object and effects of the present disclosure can be recognized and obtained especially using the components and combinations indicated in the claims. Both general explanation described above and detailed explanation described below are exemplary and explanatory and do not limit the present disclosure described in claims.

### Brief Description of Drawings

FIG. 1 is a partial cross-sectional view illustrating an example of a liquid cosmetic applicator that stores an aqueous liquid cosmetic of the present disclosure. The mechanism illustrated in FIG. 1 is an example of a rotary extension type.
FIG. 2 is a partial cross-sectional view illustrating another example of the liquid cosmetic applicator that stores the aqueous liquid cosmetic of the present disclosure. The mechanism illustrated in FIG. 2 is an example of a type of applicator with built-in cosmetic.
FIG. 3(a) is a perspective view illustrating still another example of the liquid cosmetic applicator that stores the aqueous liquid cosmetic of the present disclosure, and FIG. 3(b) is a partial cross-sectional view of the same liquid cosmetic applicator as illustrated in FIG. 3(a). The mechanism of the liquid cosmetic applicator of FIGS. 3(a) and 3(b) is an example of a collector-type applicator.

### Description of Embodiments

Embodiments of the present disclosure will be described below in detail. It should be noted that the technical scope of the present disclosure is not limited to the embodiments detailed below but includes the disclosure described in claims and equivalents thereof. The present disclosure can be implemented based on the contents disclosed in the present specification and technical common knowledge (including design matters and obvious matters) in the art.

An aqueous liquid cosmetic of the present disclosure contains at least a plant-derived pigment, a film-forming agent, a dispersant, and water, wherein the plant-derived pigment is contained in an amount of 1 to 30 mass% with respect to a total amount of the aqueous liquid cosmetic, and an intensity ratio G/D of a D band (1350 cm⁻¹) and a G band (1582 cm⁻¹) in a Raman spectrum of the plant-derived pigment is 0.5 to 10.

The plant-derived pigment used in the present disclosure is excellent in reduction of environmental load, safety, and the like, it is possible to use a black pigment or the like obtained by firing or processing a plant body such as wood or grass or its processed material as a raw material, and examples thereof include at least one kind (each alone or a mixture of two or more, the same applies hereinafter) of plant-derived charcoal such as bamboo charcoal, hemp charcoal, oak charcoal, Daimyo oak charcoal, Konara oak charcoal, oriental chestnut oak charcoal, chestnut charcoal, quercus phillyraeoides charcoal (binchotan charcoal), Japanese larch charcoal, pine charcoal, Japanese cedar charcoal, Japanese cypress charcoal, eucalyptus charcoal, mangrove charcoal, sasanqua charcoal, olive charcoal, acacia charcoal, peach black (peach core charcoal), vine black (grapevine black), activated carbon, and vegetable carbon (vegetable carbon black).

The particle shape of the plant-derived pigments is not particularly limited and may be any shape such as a spherical, granular, rod-like, needle-like, plate-like, or amorphous shape. As an average particle diameter before dispersion (at the time of blending) of the plant-derived pigment (particles) that can be used, it is possible to use the plant-derived pigment having an average particle diameter of 1 µm or more, preferably 1 to 50 µm, from the viewpoint of concern about health damage to humans from the viewpoint of a nanomaterial, the shape of the plant-derived pigment is not particularly limited, and the plant-derived pigment can have any shape such as an amorphous shape, a spherical shape, a needle shape, a plate shape, or a square shape, may be solid or hollow, and may be treated with a surface treatment agent.

In the present disclosure (including Examples), the "average particle diameter" refers to a value of a volume average particle diameter (D50) measured and calculated by a dynamic light scattering method [particle size analyzer FPAR-1000, available from OTSUKA ELECTRONICS CO., LTD] or a laser diffraction method [Microtrac MT3000II, available from MicrotracBEL Corp.].

As each plant-derived pigment such as the bamboo charcoal or the vegetable carbon used in the present disclosure, those having a G/D ratio of 0.5 to 10, which is a ratio of peak intensity of the D band and peak intensity of the G band in the Raman spectrum (Raman spectroscopic analysis), are used. The G/D ratio will be described using a Raman spectrum obtained by Raman spectroscopic analysis of a plant-derived pigment (for example, bamboo charcoal or vegetable carbon).

The G band is a peak derived from the plant-derived pigment observed around a Raman shift of 1582 cm⁻¹ in the Raman spectrum obtained by Raman spectroscopic analysis at a wavelength of 532 nm. The D band is a peak derived from a defect of amorphous carbon, graphite, or the plant-derived pigment observed around a Raman shift of 1350 cm⁻¹ in the Raman spectrum obtained by Raman spectroscopic analysis at a wavelength of 532 nm. Therefore, the larger the value of the G/D ratio, the higher the crystallinity of the plant-derived pigment, and the smaller the amount of amorphous carbon and graphite having defects contained in the plant-derived pigment.

The G/D ratio of the plant-derived pigment according to the present embodiment is 0.5 to 10, defects of amorphous carbon and graphite are small, and the crystallinity is high. Thus, the plant-derived pigment having a G/D ratio of 0.5 to 10 can have high tensile strength and high color developability.

The G/D ratio of the plant-derived pigment is preferably 0.5 to 9, more preferably 0.5 to 8, still more preferably 0.5 to 7, even more preferably 0.5 to 6, and further more preferably 0.5 to 5. When the G/D ratio of the plant-derived pigment is less than 0.5, the plant-derived pigment may not have sufficient tensile strength and high color developability. In addition, when the D/G ratio of the plant-derived pigment is more than 10, the plant-derived pigment itself may be deteriorated, which is not preferable.

In the present specification, the G/D ratio of the plant-derived pigment is a value measured by the following method.

The plant-derived pigment to be used is subjected to Raman spectroscopic analysis under the following conditions to obtain a Raman spectrum. In the Raman spectrum, the G/D ratio is calculated from the peak intensity of the D band and the peak intensity of the G band.

### (Measurement Conditions of Raman Spectroscopy)

Dispersive Raman Spectrometer manufactured by HORIBA, Ltd.: Excitation laser wavelength: 532 nm
Objective magnification × 10, light reduction filter 5%, exposure time 10 seconds × 3 times
Slit 200 µm, hole 400 µm, 300 gr/mm grating

From the viewpoint of dispersibility, the plant-derived pigment to be used preferably has a BET specific surface area of 20 to 800 m²/g, more preferably 30 to 400 m²/g.

The BET specific surface area in the present disclosure is a value measured by a BET single point method using Macsorb model HM-1208 available from MOUNTECH Co., Ltd, under pretreatment conditions of 110°C and 30 minutes using nitrogen as an adsorption gas.

As the plant-derived pigment that can be used, a plant-derived pigment in which the G/D ratio is within the range of 0.5 to 10 is used, and more preferably, it is desirable to use each plant-derived pigment having a BET specific surface area in the range of 20 to 800 m²/g. As commercially available products of plant-derived pigments falling within these ranges, for example, it is possible to use bamboo charcoal powder 5P (available from GLICO NUTRITION CO., LTD.), CHARCOAL POWDER (the raw material is Kishu binchotan charcoal, available from DAITO KASEI KOGYO CO., LTD.), fine powder binchotan charcoal powder, binchotan charcoal powder, and bamboo charcoal powder (all available from Kiriya Chemical), food additive/BAIHO A BRAND activated carbon (available from Taihei Chemical Industrial Co., Ltd.), KOELIN-663H (vegetable carbon) (available from Koel Colours Pvt. Ltd.), SpecKarre BCP3, SpecKarre BCP5, SpecKarre SuperBlnchotan, SpecKarre BCP super (all available from Spec-Chem Industry Inc.), Binchou charcoal powder pag, bamboo charcoal powder pag (available from LATEST Corporation), and the like.

The content of these plant-derived pigments is 1 to 30 mass%, preferably 5 to 20 mass%, and more preferably 5 to 15 mass% with respect to the total amount of the liquid cosmetic composition. When the content of the plant-derived pigment is less than 1 mass%, the color development is insufficient and the concealing power is also insufficient. On the other hand, when the content is more than 30 mass%, the viscosity increases so much that the application is hindered, which is not preferable.

In the present disclosure, a coloring material other than the above-described plant-derived pigment can be used in combination, as long as the effect of the present disclosure is not impaired. A pigment or a dye used in an aqueous liquid cosmetic can be used; however, in terms of color developability, combination with a pigment is particularly preferable.As pigment that can be used, specifically, at least one of inorganic pigments such as carbon black, iron oxide (red, yellow, black), titanium dioxide, titanium black (titanium oxynitride), zinc oxide, talc, chromium oxide, cobalt oxide, zinc oxide, mica titanium, bismuth oxychloride, ultramarine, and iron blue; organic pigments such as carmine, Red No. 202, Red No. 220, Red No. 226, Red No. 228, Yellow No. 401, Yellow No. 205, Yellow No. 4 Al lake, Yellow No. 203 Al lake, Red No. 40 Al lake, and Red No. 104 Al lake; whitener powders such as talc, muscovite, gold mica, red mica, black mica, synthetic mica, sericite, synthetic sericite, kaolin, silicon carbide, smectite, aluminum oxide, magnesium oxide, zirconium oxide, antimony oxide, diatomaceous earth, aluminum silicate, aluminum magnesium metasilicate, calcium silicate, barium silicate, magnesium silicate, calcium carbonate, magnesium carbonate, hydroxyapatite, boron nitride, and silicon dioxide; or sparkling powders such as titanium dioxide-coated mica, titanium dioxide-coated synthetic gold mica, titanium dioxide-coated bismuth oxychloride, iron oxide-coated mica, iron oxide-coated mica titanium, iron blue-treated mica titanium, carmine-treated mica titanium, bismuth oxychloride, fish scale foil, titanium dioxide-coated glass powder, and aluminum powder can be used in combination. The pigment including a plant-derived black pigment that can be used can be used without any limitation as long as it is used in liquid cosmetics.

Preferably, from the viewpoint of color development, it is desirable to contain a pigment selected from the following group A.

Group A: yellow iron oxide, red iron oxide, black iron oxide, titanium dioxide, Red No. 226 (CI73360, aka: Helindon Pink CN), carmine, iron blue, ultramarine, manganese violet, chromium oxide, and lake pigment.

These pigments other than the plant-derived pigments are used as coloring materials, and in particular, when at least one of the pigments of the group A is selected, it is possible to adjust a color to a gray color system, a brown color system, or Bordeaux color system in combination with the plant-derived black pigment. The content of the pigment such as the group A other than the plant-derived black pigments is preferably 0 to 20 mass%, and more preferably 0 to 15 mass% with respect to the total amount of the cosmetic composition.

Examples of the film-forming agent used in the present disclosure include those composed of a film-forming polymer, and for example, film-forming polymer emulsion particles and the like can be used. Specifically, an aqueous dispersion of a polymer insoluble in water and the like can be used.

Examples of the film-forming agent that can be used include a styrene-alkyl acrylate copolymer emulsion, a styrene-alkyl methacrylate copolymer emulsion, and a copolymer emulsion containing a component of acrylic acid, methacrylic acid, or (C1 to C4 and C8) alkyl ester thereof. It may be an emulsion of a composite polymer such as a core-shell-type polymer emulsion containing a copolymer of an ethylenically unsaturated carboxylic acid monomer and a styrene monomer and another polymer and/or copolymer.

Specific examples of the styrene-alkyl acrylate copolymer emulsion include (styrene/acrylates) copolymer. Examples of commercially available products of this polymer include YODOSOL GH41F (available from Nouryon) and DAITOSOL 5000STY (available from DAITO KASEI KOGYO CO., LTD.). Specific examples of the emulsion of the core-shell-type polymer emulsion containing a copolymer of an ethylenically unsaturated carboxylic acid monomer and a styrene monomer and another polymer and/or copolymer include EMUPOLY-CE-119N (available from Gifu Shellac Mfg. Co., Ltd.), which is an emulsion of core-shell-type copolymer of ethylhexyl acrylate/methacrylate copolymer [(acrylates/methylstyrene/styrene) copolymer ammonium]. From the viewpoint of film-forming properties and fixation of drawing lines, it is preferable to contain acrylates copolymer, and more preferably, it is desirable to use the core-shell-type polymer emulsion described above.

From the viewpoint of discharge ability, in the resin emulsion (emulsion particles) serving as the film-forming agent, the average particle diameter is preferably 50 to 300 nm, more preferably 100 to 200 nm.

In the present disclosure, by using the resin emulsion for the film-forming agent, even in the case of a plant-derived pigment having a relatively large particle diameter, a hard cake is hardly formed, storage stability is excellent, separation of a coloring material at an application tip (pen tip) hardly occurs, and a drawing line having good color developability can be applied (written) from the begining of writing.

In the film-forming agents such as emulsion particles, usually a resin component is finely dispersed in an aqueous component at a concentration of 20 to 60 mass% as a solid content.

The content of the film-forming agent to be used is 1 to 20 mass% and preferably 5 to 20 mass% with respect to the total amount of the liquid cosmetic at a concentration in terms of solid content (active ingredient concentration), and good durability against water, sweat, and sebum can be imparted to the drawing line applied to the skin in this range.

When the content of the film-forming agent is less than 1 mass%, the fixation and the water-resistant fixation are deteriorated. Meanwhile, when the content thereof is more than 20 mass%, the viscosity of the liquid cosmetic increases so much that, application of the cosmetic is difficult.

As the dispersant used in the present disclosure, a water-soluble dispersant is preferably used, and the water-soluble dispersant improves the dispersibility of a pigment such as a plant-derived black pigment to be a coloring material. Examples thereof include at least one selected from resin type dispersants such as acrylates copolymer, acrylates copolymer ammonium, (styrene/acrylates) copolymer, (styrene/acrylates) copolymer ammonium, (styrene/methylstyrene) copolymer, (acrylates/acrylamide) copolymer, (acrylates/VA) copolymer, (acrylates/VP) copolymer, (acrylates/ethylhexyl acrylate) copolymer, (acrylates/ethylhexyl acrylates/styrene) copolymer, (acrylates/octylacrylamide) copolymer, (acrylates/methylstyrene/styrene) copolymer ammonium, and (ethyl hexyl acrylate/methyl methacrylate) copolymer; surfactant-type dispersants such as polyglycerin fatty acid ester, polyoxyethylene glycerin fatty acid ester, polyoxyethylene sorbitan fatty acid ester, polyoxyethylene sorbitol fatty acid ester, polyoxyethylene hydrogenated castor oil, polyoxyalkylene sterol, polyoxyethylene alkyl ether, and polyethylene glycol fatty acid ester; and the like.

Examples of the polyglycerin fatty acid ester include glyceryl stearate, polyglyceryl isostearate-10, polyglyceryl laurate-10, polyglyceryl myristate-10, polyglyceryl oleate-10, polyglyceryl stearate-10, polyglyceryl diisostearate-10, polyglyceryl laurate-6, polyglyceryl myristate-6, and polyglyceryl palmitate-10. Examples of the polyoxyethylene glycerin fatty acid ester include coconut oil fatty acid PEG-7 glyceryl and PEG-15 glyceryl stearate.

Examples of the polyoxyethylene sorbitan fatty acid ester include PEG-20 sorbitan isostearate, polysorbate 20, polysorbate 80, PEG-5 sorbitan oleate, polysorbate 85, polysorbate 60, and polysorbate 65.

Examples of the polyoxyethylene sorbitol fatty acid ester include Sorbeth-6 laurate, Sorbeth-40 tetraoleate, and Sorbeth-60 tetraoleate.

Examples of the polyoxyethylene-hydrogenated castor oil include PEG-20 hydrogenated castor oil, PEG-30 hydrogenated castor oil, PEG-40 hydrogenated castor oil, PEG-50 hydrogenated castor oil, PEG-60 hydrogenated castor oil, PEG-80 hydrogenated castor oil, and PEG-100 hydrogenated castor oil.

Examples of the polyoxyalkylene sterol include PEG-10 phytosterol, PEG-20 phytosterol, PEG-30 phytosterol, and PEG-25 phytostanol.

Examples of polyoxyethylene alkyl ether include beheneth-10, beheneth-20, beheneth-30, ceteth-6, ceteth-7, ceteth-10, ceteth-15, ceteth-20, ceteth-23, ceteth-25, ceteth-30, ceteth-40, laureth-4, laureth-9, laureth-21, laureth-25, oleth-7, oleth-10, oleth-15, oleth-20, oleth-50, steareth-20, (C12 to C14) pareth-5, (C12 to C14) pareth-7, (C12 to C14) pareth-9, and (C12 to C14) pareth-12.

Examples of the polyoxyethylene polyoxypropylene alkyl ether include PEG-4 ceteth-10, PEG-4 ceteth-20, PEG-8 ceteth-20, PEG-4 ceteth-20, PEG-6 decyltetradeceth-20, and PEG-6 decyltetradeceth-30.

Examples of the polyethylene glycol fatty acid ester include PEG-10 stearate, PEG-25 stearate, PEG-40 stearate, PEG-45 stearate, and PEG-55 stearate.

Preferred examples thereof include those selected from acrylates copolymers, acrylates copolymer ammonium, (styrene/acrylates) copolymers, beheneth-30, ceteth-20, polyglyceryl myristate-10, polyglyceryl laurate-10, polysorbate 20, and polysorbate 80, and particularly preferably, it is desirable to use a resin type dispersant such as acrylates copolymers, acrylates copolymer ammonium, and (styrene/acrylates) copolymers which are formulated to be capable of exhibiting better durability such as water-resistant fixation and sebum resistance fixation after application by being dispersed by resin dispersion.

Examples of the dispersant that can be used include, as commercially available products, SYNTRAN EX149PE, SYNRAN 5402 (all available from Interpolymer), LUVIMER 100P (available from BASF), AMPHOMER HC (available from Nouryon N.V.), BC-5.5, BC-7, BC-10, BC-15, BC-20, BC-23, BC-25, BC-30, BC-40, BB-10, BB-20, BB-30, DDP-8, DDP-10, HCO-20, HCO-30, HCO-40, HCO-50, HCO-60, HCO-80, HCO-100, Decaglyn 1-50SV, Decaglyn 1-ISV, Decaglyn 1-L, Decaglyn 1-M, Decaglyn 1-OV, Decaglyn 1-SV, Decaglyn 2-ISV, Decaglyn 1-LVEX, Decaglyn 1-MVEX PN, Decaglyn 1-OVEX, Decaglyn 1-PVEX, Decaglyn 1-ISVEX, Decaglyn 1-SVEX, Hexaglyn 1-L, Hexaglyn 1-M, TI-10V, TL-10,TO-10MV, TO-10V, TO-106V, TO-30V, TS-10MV, TS-10V, and TS-30V (all available from Nikko Chemicals Co., Ltd.).

The content of these dispersants is preferably 5.0 to 40.0 mass%, and more preferably 7 to 30 mass% with respect to the total amount of the pigment containing the plant-derived pigment. The resin type dispersant has a solid content equivalent concentration (active ingredient concentration).

When the content of these dispersants is 5.0 mass% or more with respect to the total amount of pigments, dispersion stability of a plant-derived black pigment or the like as a coloring material is improved. On the other hand, when the content is 30 mass% or less, it is in a good viscosity range, and pigment sedimentation is improved in a case the pigment is stored in a pen-type applicator having a brush and a pen core in an application portion.

More preferably, concerning a content ratio of the film-forming agent (X) and the dispersant (Y), X/Y is 3 to 20, and more preferably 5 to 15 on a mass basis from the viewpoint of drawing line durability and stability.

In the present disclosure, in addition to the plant-derived pigment, the film-forming agent, and the dispersant, an aqueous solvent is preferably used from the viewpoint of further improving dispersion stability, low-temperature stability, applicability, and so forth.

The aqueous solvent to be used is used as a solvent of a liquid cosmetic. Examples thereof include at least one of water, a lower alcohol having 5 or less carbons, and a polyhydric alcohol.

Specific examples of the lower alcohol having 5 or less carbons include at least one of methyl alcohol (methanol), ethyl alcohol (ethanol), n-propyl alcohol, isopropyl alcohol, n-butyl alcohol, sec-butyl alcohol, tert-butyl alcohol, isobutyl alcohol, pentyl alcohol, ethylene glycol, or propylene glycol.

Examples of the polyhydric alcohol include glycerin, erythritol, threitol, arabinitol, xylitol, ribitol, propylene glycol, 1,3-butylene glycol, dipropylene glycol, pentylene glycol, dipentylene glycol, ethylene glycol, diethylene glycol, and pentaerythritol. In particular, it is desired to use ethyl alcohol (ethanol), from the perspective of safety and handleability.

The total content of the aqueous solvent to be used is preferably 45 to 85 mass%, and more preferably 50 to 80 mass%, with respect to the total amount of the liquid cosmetic, in terms of stability and dispersion stability of the plant-derived pigment, and particularly in terms of stability at low temperatures.

When the content of the aqueous solvent is 45 mass% or more, smooth writing and moisture retaining properties can be achieved, and an antiseptic effect can be slightly achieved. Meanwhile, when the content thereof is 85 mass% or less, the dispersion stability is further improved.

Water serving as a solvent used in the present disclosure can be distilled water, ion-exchanged water, purified water, pure water, or ultrapure water. In terms of further improving stability over time and dispersibility of the iron oxide pigment, the ratio of water to the aqueous solvent (water + a solvent other than water) is preferably 70 to 100 mass% (50 to 80 mass%), and particularly preferably 80 to 90 mass%.

Furthermore, the aqueous liquid cosmetic of the present disclosure can contain, in addition to the above-mentioned components, optional components used in ordinary liquid cosmetics as long as the effects of the present disclosure are not impaired. Specifically, a thickener (viscosity/rheology control agent), a preservative, a perfume, a moisturizing agent, a beauty ingredient, and a plant extract can be used, and in addition, an antioxidant, a neutralizing agent, an ultraviolet absorber, a chelating agent, a pH adjuster, or another dispersant such as polyethylene glycol alkyl ether, can be contained in an appropriate amount as long as the effects of the present disclosure are not impaired.

Examples of the thickener that can be used include polysaccharides, polymers, and/or silica thickeners. Examples of polysaccharides include starch and glycerite of starch; gums such as karaya gum (sterculia gum), tragacanth gum, arabic gum, ghatti gum, xanthan gum, guar gum, acacia gum, cellulose gum, magnesium aluminum silicate (Veegum), carrageenan, sodium alginate, agar, pectin, gelatin, and cellulose derivatives, and in the case of cellulose derivatives, for example, cellulose, carboxymethyl cellulose, hydroxyethyl cellulose, hydroxypropyl cellulose, hydroxymethyl cellulose, hydroxymethyl carboxypropyl cellulose, methyl cellulose, ethyl cellulose, sulfated cellulose, cellulose oxide, and crystalline cellulose can be used. In addition, natural and synthetic clays such as bentonite and hectorite clays and mixtures thereof can be used as examples.

Examples of the preservatives that can be used include an iodopropargyl compound, sodium pentachlorophenol, 1,2-benzisothiazolin-3-one, 2,3,5,6-tetrachloro-4(methylsulfonyl)pyridine, paraoxybenzoic acid ester, phenol, sodium benzoate, sodium dehydroacetate, potassium sorbate, morpholine, cresol, methylisothiazolinone, chloromethylisothiazolinone, octylisothiazolinone, dichlorooctylisothiazolinone, hexahydro-1,3,5-tris(2-hydroxyethyl)-1,3,5-triazine, 2-bromo-2-nitropropane-1,3-diol, sodium 2-pyridinethiol-1-oxide, sodium pyrithione, 2-(4-thiozolyl)benzimidazole, 4-terpineol, 1,8-cineole, thymol, diisothiocyanate, eucalyptus oil, longifolene, isopropylmethylphenol, 2-methyl-4-isothiazolin-3-one, citral, eugenol, allyl isothiocyanate, d-limonene, tannic acid, benzalkonium chloride, glyceryl caprylate, glycerin fatty acid ester, chlorphenesin, salicylic acid, ethyl paraoxybenzoate, butyl paraoxybenzoate, propyl paraoxybenzoate, methyl paraoxybenzoate, bisabolol, hinokitiol, phenylethyl alcohol, phenethyl alcohol, phenoxyethanol, butylparaben, propylparaben, benzalkonium chloride, methyl paraben, 2-(4-thiazolyl)benzimidazole, 1,2-propanediol, 1,2-pentanediol, 1,2-hexanediol, 1,2-octanediol, and ethylhexylglycerin.

In the present disclosure, since a plant-derived pigment such as charcoal having a weaker antiseptic effect than a pigment such as carbon black is used, it is possible to obtain an aqueous liquid cosmetic capable of satisfying an antiseptic standard and highly achieving the effects of the present disclosure by adjusting an antiseptic design or the like in which each of the antiseptics described above, particularly preferably, 1,2-pentanediol, 1,2-hexanediol, 1,2-octanediol, and the like is set to preferably 0.5 to 10 mass%, more preferably 1 to 5 mass% with respect to the total amount of the liquid cosmetic.

Examples of perfumes that can be used include at least one selected from the group consisting of anethol, cineole, basil, clary sage, citral, α-pinene, β-pinene, γ-terpinene, nonanol, octanol, citronellal, ionone, methyl ionone, lavender, marjoram, oregano, patchouli, rosemary, sage, rose wood, clove, cinnamon, thyme, eugenol, linalool, tea tree, α-terpineol, citronellol, limonene, α-terpinene, acetylcedrene, α-terpenyl acetate, cedrol, lavandulyl acetate, 1,8-cineole, terpinen-4-ol, myrcene, linalyl acetate, benzyl acetate, β-caryophyllene, squaleol, geranyl acetate, neryl acetate, dimethyl tetrahydrobenzaldehyde, Ambroxan, geranyl nitrile, bacdanol, acetonaphthone, hexamethylhexahydrocyclopentane, benzopyran, coumarin, γ-phenethyl alcohol, lilial, vanillin, methyl dihydrojasmonate, peppermint oil, sweet orange oil, ceder wood, lavandin oil, geranium oil, lemon oil, citronella oil, and lime oil.

Among them, at least one selected from the group consisting of anethol, cineral, citral, lavender, linalool, tea tree, citronellal, and geranium is preferable.

Examples of the moisturizing agent, beauty ingredient, and the like that can be used include Na hyaluronate, panthenol, oligopeptide, sage leaf extract, aloeferox leaf extract, succinoyl atelocollagen, deep water, Mimasaka hot spring water, marine cholesterol, and hydrolyzed silk.

Examples of the plant extract that can be used include shikonin extract, grape leaf extract, uncaria gambir extract, althea extract, aloe extract, ginkgo biloba leaf extract, urtica thunbergiana extract, artemisia capillaris extract, foeniculum vulgare (fennel) fruit extract, malva sylvestris (mallow) extract, rose fruit extract, scutellaria baicalensis extract, phellodendron bark extract, coptis japonica extract, hypericum erectum extract, seaweed extract, artemisia princeps extract, chamomile extract, cinchona extract, cucumber extract, lonicera japonica extract, sophora flavescens extract, sasa veitchii leaf extract, gentiana lutea extract, geranium thunbergii extract, arctium lappa root extract, symphytum officinale extract, crataegus cuneata fruit extract, lentinus edodes extract, rehmannia chinensis extract, perilla extract, linden extract, houttuynia cordata extract, sanguisorba officinalis root extract, birch extract, equisetum arvense extract, hedera helix (ivy) extract, achillea millefolium extract, mulberry bark extract, jujube extract, wild thyme extract, green tea extract, clove extract, calendula officinalis extract, angelica acutiloba extract, bitter orange extract, tomato extract, sambucus nigra extract, hamamelis virginiana (witch hazel) extract, eriobotrya japonica leaf extract, tussilago farfara extract, aesculus hippocastanum (horse chestnut) extract, melissa officinalis extract, prunus persica (peach) leaf extract, saxifraga sarmentosa extract, lavender extract, lemon extract, royal jelly extract, chestnut extract, rose extract (rosa centifolia flower extract), apple extract (malus domestica fruit cell culture extract), swertia japonica extract, pinus sylvestris cone extract, chamomilla recutita (matricaria) flower extract, camellia sinensis leaf extract, prunus lannesiana flower extract, panax ginseng root extract, brown algae extract, rosa canina fruit extract, prunus persica (peach) leaf extract, olea europaea (olive) leaf extract, and rosemary extract. Each of those plant extracts may be used alone, or two or more thereof may be used in mixture.

In the aqueous liquid cosmetic of the present disclosure, from the viewpoint of liquid outflow property and dispersion stability of the cosmetic, preferably, it is desirable to adjust a viscosity range of each viscosity at a temperature of 25°C, a shear rate of 192 (s⁻¹) (50 rpm), and a shear rate of 38.3 [s⁻¹] (10 rpm) from the mounting type and application type of the liquid cosmetic applicator.

In the liquid cosmetic applicator of a collector type or a cotton-pad type, when a viscosity value at a shear rate of 192 [s⁻¹] (50 rpm) is 15 mPa·s or less, and more preferably 2 to 8 mPa·s, it is possible to write a fresh drawing line with excellent discharge ability and good color developability.

In a direct liquid storage type (piston extrusion type, valve type), when the viscosity value at a shear rate of 38.3 [s⁻¹] (10 rpm) is 10 mPa·s or more and 100 mPa·s or less, stable discharge ability is maintained, color development is good, and a drawing line can be written without smearing.

It is not preferable that the viscosity value is more than the upper limit (15 mPa·s, 100 mPa·s) because the outflow property of the aqueous liquid cosmetic is significantly deteriorated.

In the present disclosure, an aqueous liquid cosmetic having a good formulation design without a difference in concentration between an upper portion and a lower portion of the liquid cosmetic can be obtained even with the above-described low-viscosity formulation.

The viscosity ranges can be adjusted by suitably combining the raw materials such as the plant-derived pigment, the film-forming agent, the dispersant, and the aqueous solvent to be used and combining their contents in suitable ranges, or by a suitable dispersion method.

In the present disclosure, regarding the viscosity measurement conditions (including Examples described below), specifically, the resultant cosmetic composition was measured with an ELD-type viscometer available from Toki Sangyo Co., Ltd using a standard rotor: shear rate: 192 [s⁻¹] (50 rpm) or 38.3 [s⁻¹] (10 rpm) at a temperature of 25°C.

The aqueous liquid cosmetic of the present disclosure preferably has a pH (25°C) in a range of 6 to 9 when measured using a glass electrode, from the viewpoint of improving solubility and suppressing skin irritation. The pH is adjusted by adjusting types and amounts of the components, and using the above-mentioned pH adjusting agent, or the like.

In the aqueous liquid cosmetic of the present disclosure, the plant-derived pigment, the film-forming agent, the dispersant, the aqueous solvent, and other components can be prepared under suitable dispersion conditions using, for example, a disperser such as a homomixer, a sand mill, an ultrasonic homogenizer, or a high-pressure homogenizer.

Preferably, when a (multiple type) ultrasonic homogenizer or a high-pressure homogenizer is used as the dispersion machine, as the dispersion conditions, for example, in the case of an ultrasonic homogenizer, the frequency is suitably adjusted, and in the case of a high-pressure homogenizer, a pressure range is suitably adjusted, whereby optimum dispersion can be performed.

The obtained aqueous liquid cosmetic containing the plant-derived pigment is mounted on the liquid cosmetic applicator of a collector type or a cotton-pad type or the liquid cosmetic applicator of a direct liquid storage type (piston extrusion type, valve type), and, for example, the aqueous liquid cosmetic can be stored (filled) in a pen-type liquid cosmetic applicator (container) having a brush or a pen core as an application portion and provided for use.

The liquid cosmetic applicator that can be used is not particularly limited as long as it is a liquid cosmetic applicator of each mounting type, which is equipped with a brush or a pen core for, for example, an eyeliner or an eyebrow pen.

Preferred examples of the liquid cosmetic applicator include an applicator having, as application means, a brush (brush pen) or a pen core for an eyeliner or an eyebrow pen, or an application body formed of rubber, an elastomer, or a closed-cell foam having restorability, and having a container for charging the liquid cosmetic.

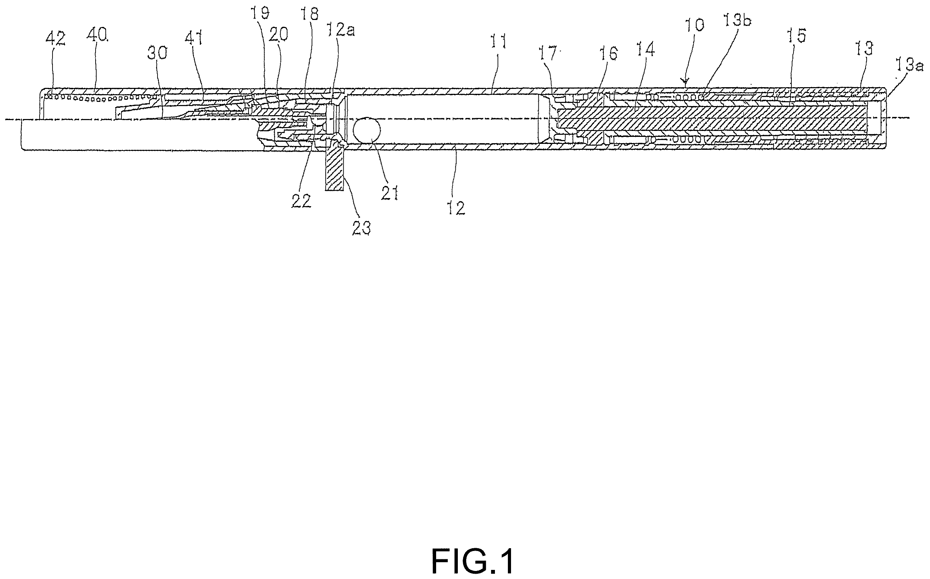

Specifically, as illustrated in FIGS. 1, 2, 3(a), and 3(b), it is desirable to use a liquid cosmetic applicator which is excellent in usability, convenience, and applicability and has a mechanism of a rotary extension type, a mechanism of a type of applicator with built-in cosmetic, or a mechanism of a collector-type applicator.

As illustrated in FIG. 1, a liquid cosmetic applicator of a rotary extension type includes an application portion 30 formed of a brush (brush pen) provided in front of a cosmetic container 11 serving as a reservoir configured to discharge, by a liquid pressing mechanism 10, the aqueous liquid cosmetic containing the plant-derived pigment of the present disclosure (hereinafter referred to simply as "liquid cosmetic") contained in front of the liquid pressing mechanism 10.

The liquid pressing mechanism 10 has such a configuration that a feeding member 13 disposed at a rear end portion of a barrel body 12 is relatively rotated with respect to the barrel body 12 in a circumferential direction to feed the liquid cosmetic in the container (reservoir) 11, and then the liquid cosmetic is supplied to the application portion 30.

The liquid pressing mechanism 10 of the applicator has the feeding member 13 rotatably fitted to the rear end of the barrel body 12; a driving cylinder 15 configured to transmit the rotational force of the feeding member 13 by a user to a screw rod 14; a screw body 16 fixed to the barrel body 12 and screwed with the screw rod 14; the screw rod 14 rotatably engaged with a piston body 17 at its tip; and the piston body 17 configured to slide in the reservoir 11 of the barrel body 12. The rotation of the feeding member 13 is transmitted to the screw rod 14 via the driving cylinder 15, and the screw rod 14 and the piston body 17 are advanced through a female screw of the nut-like screw body 16 by the rotation of the screw rod 14, to thereby feed the liquid cosmetic from the reservoir 11 to the application portion 30.

As illustrated in FIG. 1, an operating portion of the feeding member 13 is rotatably fitted into the rear end portion of the barrel body 12 and is exposed therefrom, the operating portion having a cylindrical shape closed by fitting a crown 13a into the rear end thereof. The driving cylinder 15 is fitted into the feeding member 13 and fixed in the rotating direction. The screw body 16 is mounted in the driving cylinder 15 to be fixed in the rotating direction and to be relatively movable in the axial direction. Reference numeral 13b denotes a spring member that rearwardly biases the feeding member 13 serving as a rotating body.

In this applicator, a sealing portion 18, a joint member 19, a front barrel 20, and the application portion 30 are fitted into the front end portion 12a of the barrel body 12. The liquid cosmetic is stored in the reservoir 11 of the barrel body 12, and the liquid cosmetic fed out from the reservoir 11 is discharged to the application portion 30 through a flow path in the joint member 19 to be applicable. The applicator is configured such that, after use, a cap 40 can be attached to the front barrel 20 to cover the application portion 30 and the front barrel 20.

In FIG. 1, reference numeral 21 denotes a stirring ball configured to stir the liquid cosmetic in the reservoir 11 by reciprocating motion, and reference numeral 22 denotes a sealing ball. Reference numeral 41 denotes an inner cap in the cap 40, and reference numeral 42 denotes a spring for biasing the inner cap rearward. The stirring ball 21 may be omitted.

Reference numeral 23 denotes a stopper having a ring-shaped portion mounted between the rear end of the front barrel 20 and the front surface of a stepped portion of the front end portion 12a of the barrel body 12, to locate the sealing portion 18, the joint member 19, the front barrel 20, and the application portion 30 at a position for closing the flow path of the liquid cosmetic toward the application portion 30 when not in use. In the stopper 23, a part of a ring-shaped portion is cut off, and a knob piece is integrally formed on the opposite side of the cut-off portion. When the knob piece is pulled, the diameter of the ring-shaped portion is enlarged from the cut-off portion, to be removed from between the rear end of the front barrel 20 and the front end portion 12a of the barrel body 12.

As illustrated in FIG. 1, when not in use, the sealing ball 22 fits into and seals the inner diameter portion of the sealing portion 18 serving as a sealing ball receiver, and thus the liquid cosmetic does not flow toward the application portion 30. At the time of use, when a user pulls out the stopper 23 from the barrel body 12 and pushes the front barrel 20 to the rear end side, a small-diameter portion of the rear end of the joint member 19 abuts against the sealing ball 22, and the sealing ball 22 is removed from the inner diameter portion of the sealing portion 18 and enters into the reservoir 11. Then, the liquid cosmetic in the reservoir 11 flows into a liquid flow channel of the application portion 30 from an inner diameter portion of the joint member 19 and is supplied into the application portion 30 from its inside. Then, the liquid cosmetic is applicable to a target portion.

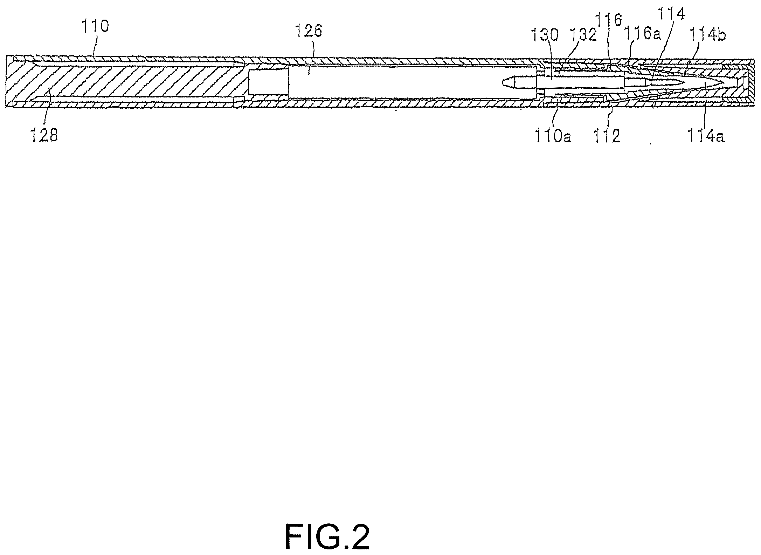

As illustrated in FIG. 2, a liquid cosmetic applicator that is a type of applicator with built-in cosmetic has a barrel 110 including an inner cotton 126 impregnated with a cosmetic; an application portion 114 that is provided at a barrel tip 110a and is configured to apply the cosmetic to a target; and a holding member 116 that covers the outer periphery of the side of the barrel 110 (base side) of the application portion 114 except for a tip 114a of the application portion 114. The applicator with built-in cosmetic has a structure in which the cosmetic inside the barrel 110 is supplied to the application portion 114, and has a cap 112 removably attached to the barrel tip 110a to cover the application portion 114 and the holding member 116.

The tip 114a of the application portion 114 is sharpened. The application portion 114 has a brush-like shape in which fibers are bundled. Specifically, the application portion 114 is formed by arranging (sharpening) a plurality of resin-made fiber bundles (specific examples: the material of the fibers is made of polybutylene terephthalate (PBT) and the fiber thickness is 0.1 to 0.15 mm) in a brush-like shape to thereby taper the tip 114a, and integrating the rear end portion by heat welding and expanding the diameter in a flange-like shape. Alternatively, the application portion 114 may be formed by solidifying other fibers.

As illustrated in FIG. 2, the applicator with built-in cosmetic of this embodiment includes the inner cotton 126 of an ink absorber from the central portion to the tip 110a of the tip portion in the barrel 110, and the inner cotton 126 is sealed and supported by a tail plug 128 inserted from the rear end of the barrel 110.

An ink relay core 130 made of an open-cell foam is disposed in the opening of the barrel tip 110a. The tail end of the relay core 130 is fitted into the tip of the inner cotton 126, while the tip thereof is fitted into the tail end portion of the application portion 114, thereby guiding the ink occluded in the inner cotton 126 to the application portion 114. The relay core 130 is mounted via a substantially cylindrical support 132 in the barrel tip 110a that is stepped relative to the main body and has a slightly reduced diameter (reduced diameter by the thickness of the cap 112). The cylindrical rear end portion of the holding member 116 is fitted between the outer periphery of the support 132 and the inner periphery of the barrel tip 110a.

The tip of the holding member 116 is located forward of the barrel tip 110a and covers the outer peripheral surface 114b of the application portion 114, and the holding member outer peripheral surface 116a is tapered in a shape of a conical side surface or a tapered shape.

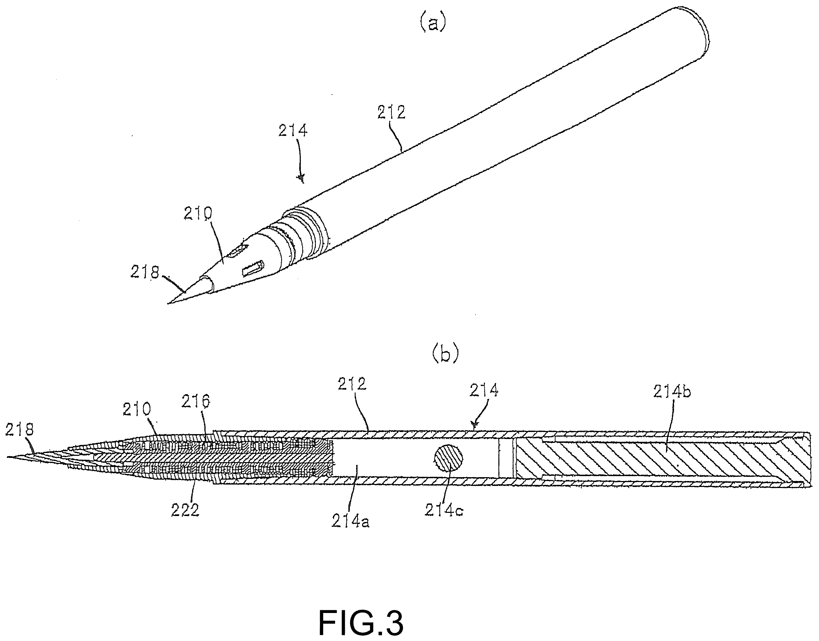

As illustrated in FIG. 3(a), the liquid cosmetic applicator that is a collector-type applicator has a barrel 214 in which a front barrel 210 and a barrel body 212 at the rear side of the front barrel 210 are fitted into each other. As illustrated in FIG. 3(b), a collector 216, which is formed in a comb-like shape in an embodiment in which a plurality of sheet portions are arranged in the axial direction, is disposed in the front portion of the barrel 214, and an application liquid is stored in a storage space 214a in the rear portion in the barrel 214.

The barrel body 212 at the rear portion of the barrel 214 is formed in a pipe shape that communicates with the inside and is opened in the front and rear direction. A tail plug 214b is fitted to a rear portion of the barrel body 212 that is also a rear portion of the barrel to close the rear portion of the barrel body 212. A space in the barrel 214 (also a space in the barrel body 212) sandwiched between a front end of the tail plug 214b and a rear end of the collector 216 is the storage space 214a.

In the storage space 214a, an impregnation body such as an inner cotton is not disposed, but an application liquid is directly stored, and a stirring body (e.g., ball) 214c configured to stir the application liquid is disposed.

The front barrel 210, the barrel body 212, the collector 216, and the cap may be resin molded products. A ball material made of a metal or a resin can be used for the stirring body 214c.

The collector 216 is covered and held by the front barrel 210 and the barrel body 212.

A writing portion 218 formed of a brush body having a tapered shape protrudes from an opening at the front end portion of the front barrel 210, and a cap covering the writing portion 218 is removably fitted to the front barrel 210. The front barrel 210 has a substantially conical side surface shape and is formed to be tapered, and the front barrel 210 is desirably formed such that the tip angle thereof is substantially equal to the tip angle of the writing portion 218.

The writing portion 218 is a tapered brush body made of resin fibers, natural fiber bundles, or a resin porous body. The rear end portion of the writing portion 218 has an enlarged diameter in a flange shape. This enlarged portion is engaged with the inside of the front barrel 210 and prevents the front barrel 210 from being removed. The writing portion 218 is suitably a brush body, but any other application body configured to apply an application liquid can be used.

In the cap, a cup-shaped inner cap configured to cover the writing portion 218 to enhance airtightness is disposed to be movable back and forth, and a spring for biasing the inner cap rearward is disposed.

A bellows-like collector 216 is disposed behind the writing portion 218 in the inside of the hollow tapered front barrel 210, and a core 222 penetrates through the hollow portion of the collector 216 and is disposed therein. The core 222 serving as the relay core can be formed of a capillary member such as a resin fiber bundle, a natural fiber bundle, or a resin porous body.

In the core 222, the core 222 does not protrude from the rear end portion of the collector 216 into the storage space 214a of the barrel 214 (see FIG. 3(b)). The rear end surface of the core 222 substantially matches the rear end surface of the collector 216. By matching the core 222, the rear end of the core 222 does not protrude into the storage space 214a, and the volume in the storage space 214a can be ensured. Since the rear end of the core 222 does not protrude into the storage space 214a, when the stirring body 214c is provided in the storage space 214a, the stirring body 214c does not collide with the core 222 and does not deform the core 222 even if the stirring body 214c moves in the storage space 214a. Therefore, the application liquid can sufficiently penetrate through the core.

As the liquid cosmetic applicator on which the aqueous liquid cosmetic of the present disclosure is mounted, a direct-liquid type liquid cosmetic applicator is preferable, and a collector type liquid cosmetic applicator is more preferable. When the relay core to be used has a porosity of 30% or more, clogging of the coloring material and the like hardly occur, and excellent discharge ability is obtained. In addition to the direct-liquid type liquid cosmetic applicator, a cotton-pad type, a direct liquid storage type (piston extrusion, with stirring ball), and a direct liquid storage type (valve type) can be used, and a "brush", a "fiber bundle core", and a "spatula" can be used in the application portion.

The liquid cosmetic applicator of the above embodiment has been described with reference to, for example, a liquid cosmetic applicator of liquid eyeliner or liquid eye shadow that is a cosmetic composition as the aqueous liquid cosmetic containing the plant-derived pigment of the present disclosure. However, the present disclosure is not limited thereto, and the present disclosure can also be applied to an eyebrow applicator for drawing lines on eyebrows, an applicator for mascara, and for drawing a line on the skin.

The applicator of rotary extension type illustrated in FIG. 1 is used as a liquid pressing mechanism of the liquid cosmetic applicator of the above embodiment, but a liquid cosmetic applicator of knock extension type may be used.

In the aqueous liquid cosmetic of the present disclosure configured as described above, by suitably combining at least 1 to 30 mass% of a plant-derived pigment in which the G/D ratio is 0.5 to 10, a film-forming agent, a dispersant, and preferably each raw material such as an aqueous solvent, the aqueous liquid cosmetic is excellent in color developability and dispersion stability even when the plant-derived pigment is used, the aqueous liquid cosmetic can be discharged well even when mounted on an applicator for makeup cosmetics such as a direct-liquid eyeliner, an eyeshadow, an eyebrow for drawing lines on eyebrows, and a mascara as well as a cotton-pad-type liquid cosmetic applicator, and can draw a drawing line having high color development, and an aqueous liquid cosmetic excellent in durability such as water-resistant fixation can be obtained. Thus, even when the aqueous liquid cosmetic containing the plant-derived pigment of the present disclosure is stored in a container such as a liquid cosmetic applicator to be used for a long period of time, dispersibility and durable stability in the container are maintained for a long period of time since a plant-derived pigment such as charcoal is used unlike iron oxide pigments and the like in which aggregation, sedimentation and the like occur; therefore, for example, even in an applicator container of an eyeliner using a brush (brush pen) for the application portion 30 as shown in FIG. 1, color separation hardly occurs at the tip of the brush, and an aqueous liquid cosmetic suitable for a liquid cosmetic such as an eyeliner liquid excellent in visibility such as vivid color development, concealing properties, and mat feeling can be obtained.

Although the applicator container as illustrated in FIG. 1 or 3 is provided with a stirring body, since the plant-derived pigment is used, a user need not perform stirring, and no color change occurs at the time of application.

### Examples

The present disclosure will next be described in further detail with reference to Examples and Comparative Examples, but the present disclosure is not limited to the following Examples.

### [Examples 1 to 15 and Comparative Examples 1 to 3]

A cosmetic composition (blending unit: mass%, total amount: 100 mass%) to be an aqueous liquid cosmetic having a formulation shown in Table 1 below was prepared by the following method, and the viscosity value of each liquid cosmetic was measured by the above measurement method, and the measurement of pH, color developability, drawing line durability A (water-resistant fixation), drawing line durability B (skin grease resistance), product durable stability, and liquid cosmetic discharge ability were evaluated by the following evaluation method.

The aqueous liquid cosmetic of Examples 1 to 13 was evaluated for color developability, drawing line durability A (water-resistant fixation), drawing line durability B (skin grease resistance), product durable stability, and liquid cosmetic discharge ability using a direct-liquid (collector) type liquid cosmetic applicator (eyeliner) of FIG. 3, and the aqueous liquid cosmetics of Examples 14 and 15 were evaluated for color developability, drawing line durability A (water-resistant fixation), drawing line durability B (skin grease resistance), product durable stability, and liquid cosmetic discharge ability using a feeding type liquid cosmetic applicator (eyeliner) of FIG. 1 in the same manner as in Examples 1 to 13.

The results are shown in Table 1 below.

### (Method for preparing aqueous liquid cosmetic)

A cosmetic composition as an aqueous liquid cosmetic having the formulation shown in Table 1 below was prepared by a typically used procedure. Specifically, a plant-derived pigment as a coloring material and a dispersant were added to ion exchanged water as a vehicle and were dispersed using LABSTAR (available from Ashizawa Finetech Ltd.) as a dispersing machine. Then, other components were added to the dispersion, followed by mixing to prepare a cosmetic composition.

### (Method of measuring viscosity)

The viscosity of each of the aqueous liquid cosmetics obtained by the above-described method was measured with an ELD-type viscometer available from Toki Sangyo Co., Ltd using a standard rotor: shear rate: 192 [s⁻¹] (50 rpm) or a standard rotor: shear rate: 38.3 [s⁻¹] (10 rpm) at 25°C.

### (Method of measuring pH)

The pH of each of the aqueous liquid cosmetics obtained by the above method was measured at 25°C with a glass electrode pH meter.

### (Method of evaluating color developability)

The obtained aqueous liquid cosmetic was charged in the collector type eyeliner of FIG. 3 and was applied to the skin, and the color developability was evaluated according to the following evaluation criteria.

### Evaluation criteria:

A: Very good color development.
B: Although the skin slightly remains visible, the color development is good.
C: The color development is not so good, and the skin remains visible.
D: Poor color development, and the skin remains visible.

### [Method for evaluating drawing line durability A (water-resistant fixation)]

The obtained aqueous liquid cosmetic was charged in the collector type eyeliner of FIG. 3, applied to a forearm portion, and then poured with running water. The applied surface was rubbed with the ball of a finger to evaluate water-resistant fixation according to the following evaluation criteria.
A: The drawing line is not changed by rubbing with the ball of a finger.
B: A slight change is observed in the drawing line by rubbing with the ball of a finger.
C: A change is observed in the drawing line by rubbing with the ball of a finger.
D: The drawing line is erased by rubbing with the ball of a finger.

### [Method for evaluating drawing line durability B (sebum resistance fixation)]

The obtained aqueous liquid cosmetic was charged in the collector type eyeliner of FIG. 3, applied to a forearm portion, then applied with artificial sebum, and rubbed with the ball of a finger to evaluate the sebum resistance fixation according to the following evaluation criteria.
A: The drawing line is not changed by rubbing with the ball of a finger.
B: A slight change is observed in the drawing line by rubbing with the ball of a finger.
C: A change is observed in the drawing line by rubbing with the ball of a finger.
D: The drawing line is erased by rubbing with the ball of a finger.

### (Method for evaluating product durable stability)

The collector type eyeliner of FIG. 3 was filled with the obtained aqueous liquid cosmetic, and the state after 1 month passed at 50°C was optically evaluated according to the following evaluation criteria.

### Evaluation criteria:

A: Pigment sedimentation was not observed.
B: Pigment sedimentation was observed, and the precipitate was easily loosened.
C: Pigment sedimentation was observed, and the precipitate was not easily loosened.
D: Pigment sedimentation was observed, and the precipitate was hard and was not loosened.

### (Method for evaluating liquid cosmetic discharge ability)

The obtained aqueous liquid cosmetic was charged in the collector type eyeliner of FIG. 3 and was applied to the skin, and the liquid cosmetic discharge ability was evaluated according to the following evaluation criteria.

### Evaluation criteria:

A: There is no problem in the flow rate, it is moist, and the application portion is also good
B: Although a decrease in flow rate is observed, there is no problem in application, and the application portion is not chipped
C: The flow rate decreases, and slight chipping is observed in the application portion
D: A remarkable decrease in flow rate is observed, it is dry, and the application portion cannot be visually recognized.

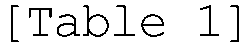

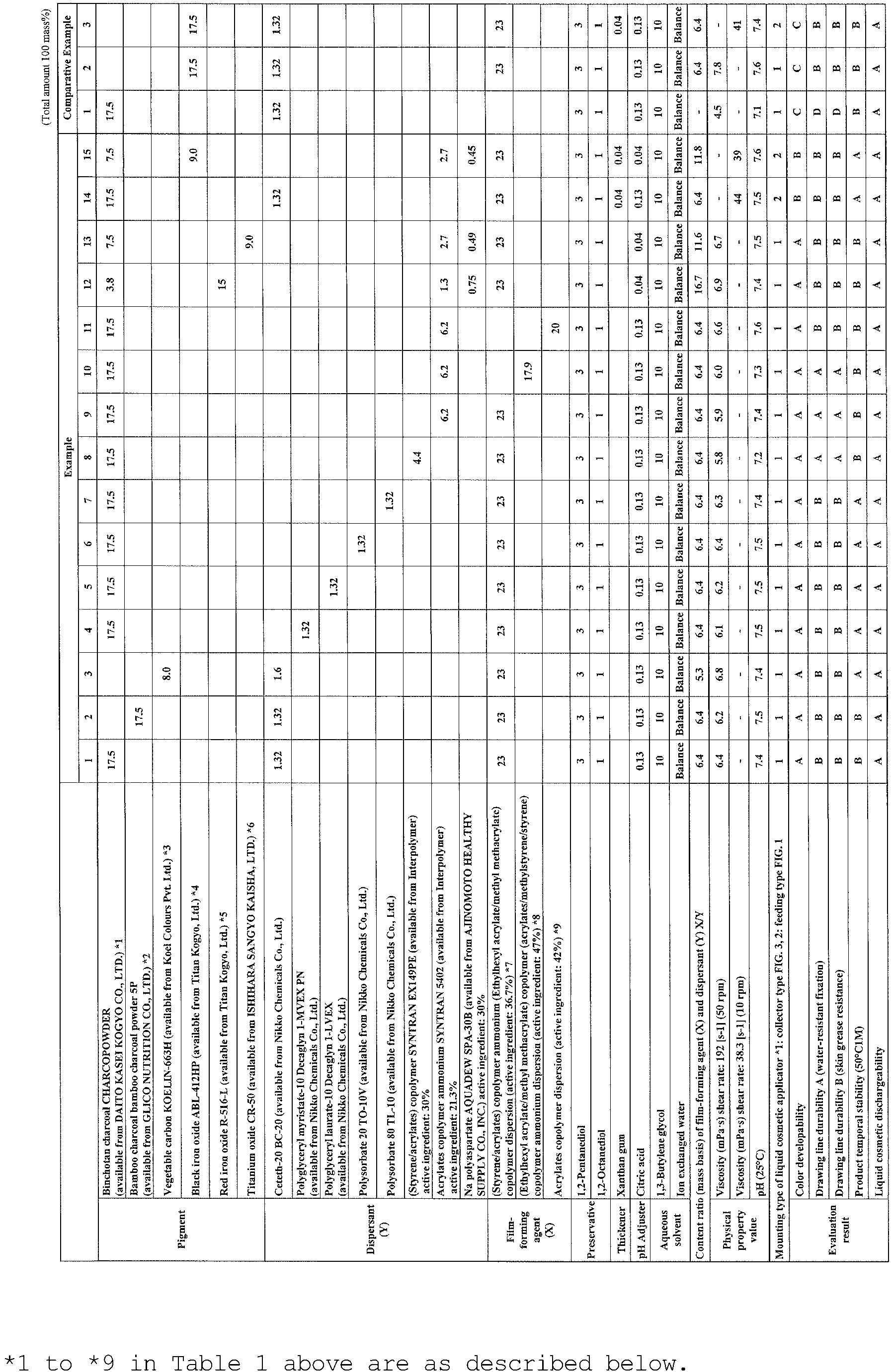

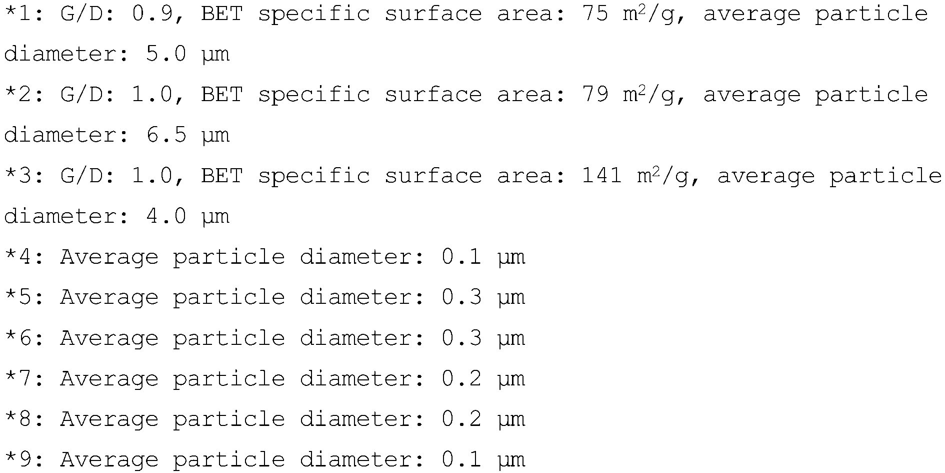

As is apparent from the results in Table 1 above, the aqueous liquid cosmetic using the collector type liquid cosmetic applicator in Examples 1 to 13 and the aqueous liquid cosmetic using the feeding type liquid cosmetic applicator in Examples 14 and 15, which fall within the scope of the present disclosure, were found to be excellent in color developability, drawing line durability (water-resistant fixation and skin grease resistance), product durable stability, and liquid cosmetic discharge ability, as compared with Comparative Examples 1 to 3 outside the scope of the present disclosure.

Further, in Examples 14 and 15 described above, the color developability, the drawing line durability (water-resistant fixation and skin grease resistance), the product durable stability, and the liquid cosmetic discharge ability were evaluated using the collector type liquid cosmetic applicator in FIG. 3 by adjusting the viscosity instead of the feeding type, so that it was confirmed that each evaluation result similar to the case of using the feeding type was obtained.

Furthermore, in Examples 1 to 13 described above, the color developability, the drawing line durability (water-resistant fixation and skin grease resistance), the product durable stability, and the liquid cosmetic discharge ability were evaluated using the feeding type liquid cosmetic applicator in FIG. 1 by adjusting the viscosity instead of the collector type, so that it was confirmed that each evaluation result similar to the case of using the collector type was provided.

### Industrial Applicability

When a plant-derived pigment excellent in reduction of environmental load, safety is used, it is possible to provide an aqueous liquid cosmetic which is excellent in color developability and dispersion stability, easy to discharge, when mounted on an applicator for makeup cosmetics such as a direct-liquid eyeliner, an eyeshadow, an eyebrow for drawing lines on eyebrows, and a mascara as well as a cotton-pad-type liquid cosmetic applicator. Further, it is possible to draw a drawing line having high color development with drawing line durability.

### Reference Signs List

- 10: Liquid pressing mechanism
- 12: Barrel body
- 13: Feeding member
- 17: Piston body
- 18: Sealing portion
- 19: Joint member
- 20: Front barrel
- 30: Application portion (brush)

## Claims

1. An aqueous liquid cosmetic comprising at least a plant-derived pigment, a film-forming agent, a dispersant, and water,
wherein the plant-derived pigment is contained in an amount of 1 to 30 mass% with respect to a total amount of the aqueous liquid cosmetic, and an intensity ratio G/D of a D band (1350 cm⁻¹) and a G band (1582 cm⁻¹) in a Raman spectrum of the plant-derived pigment is 0.5 to 10.

2. The aqueous liquid cosmetic according to claim 1, wherein the plant-derived pigment has a BET specific surface area of 20 to 800 m²/g.

3. The aqueous liquid cosmetic according to claim 1 or 2, further comprising a pigment selected from the following group A:
group A: yellow iron oxide, red iron oxide, black iron oxide, titanium dioxide, Red No. 226, carmine, iron blue, ultramarine, manganese violet, chromium oxide, and lake pigment.

4. The aqueous liquid cosmetic according to claim 1 or 2, wherein the film-forming agent is a resin emulsion.

5. A liquid cosmetic applicator comprising the aqueous liquid cosmetic according to claim 1 or 2.
